# EUROPEAN PATENT SPECIFICATION

(11) **EP 3 224 348 B1**
(45) Date of publication and mention of the grant of the patent: **14.07.2021**
(21) Application number: 15805415.5
(22) Date of filing: 26.11.2015
(51) Int. Cl.: C12N 5/0775, C12N 5/0784

(54) **SECRETOMES AND METHOD FOR PRODUCING SECRETOMES**
SEKRETOME UND VERFAHREN ZUR HERSTELLUNG VON SEKRETOMEN
SÉCRÉTOMES ET LEURS PROCÉDÉS DE PRODUCTION

(30) Priority: 27.11.2014 WO PCT/EP2014/075875; 02.07.2015 CH 956152015
(43) Date of publication of application: 04.10.2017
(73) Proprietor: Med Cell Bahamas Ltd., Nassau (BS)
(72) Inventor: KELLNER, Steven James, 9542 Zuzwil SG (CH)
(74) Representative: Rentsch Partner AG
(86) International application number: PCT/EP2015/077760
(87) International publication number: WO 2016/083500

(56) References cited:
- EP-A1- 2 607 478
- WO-A1-2013/150303
- CHING-PING CHANG ET AL: "Hypoxic preconditioning enhances the therapeutic potential of the secretome from cultured human mesenchymal stem cells in experimental traumatic brain injury", CLINICAL SCIENCE, vol. 8, no. 3, 5 October 2012 (2012-10-05) , pages 4A1.1-176, XP055161098, ISSN: 0143-5221, DOI: 10.1016/j.neulet.2008.03.096
- LEI CHEN ET AL: "Conditioned Medium from Hypoxic Bone Marrow-Derived Mesenchymal Stem Cells Enhances Wound Healing in Mice", PLOS ONE, vol. 9, no. 4, 29 April 2014 (2014-04-29), page e96161, XP055161042, DOI: 10.1371/journal.pone.0096161
- SUDHIRH RANGANATH ET AL: "Harnessing the Mesenchymal Stem Cell Secretome for the Treatment of Cardiovascular Disease", CELL STEM CELL, vol. 10, no. 3, 2 March 2010 (2010-03-02), pages 244-258, XP028402789, ISSN: 1934-5909, DOI: 10.1016/J.STEM.2012.02.005 [retrieved on 2012-02-14]
- KUPCOVA SKALNIKOVA HELENA ED - SALGADO ANTONIO ET AL: "Proteomic techniques for characterisation of mesenchymal stem cell secretome", BIOCHIMIE, vol. 95, no. 12, 20 July 2013 (2013-07-20) , pages 2196-2211, XP028768034, ISSN: 0300-9084, DOI: 10.1016/J.BIOCHI.2013.07.015
- HYUN OK KIM ET AL: "Mesenchymal stem cell-derived secretome and microvesicles as a cell-free therapeutics for neurodegenerative disorders", TISSUE ENGINEERING AND REGENERATIVE MEDICINE, vol. 10, no. 3, 25 May 2013 (2013-05-25), pages 93-101, XP055161063, ISSN: 1738-2696, DOI: 10.1007/s13770-013-0010-7
- ZORAN IVANOVIC ET AL: "Primitive human HPCs are better maintained and expanded in vitro at 1 percent oxygen than at 20 percent", TRANSFUSION, vol. 40, no. 12, 1 December 2000 (2000-12-01), pages 1482-1488, XP055161089, ISSN: 0041-1132, DOI: 10.1046/j.1537-2995.2000.40121482.x
- WANG J A ET AL: "Anoxic preconditioning: A way to enhance the cardioprotection of mesenchymal stem cells", INTERNATIONAL JOURNAL OF CARDIOLOGY, ELSEVIER SCIENCE PUBLISHERS, AMSTERDAM, NL, vol. 133, no. 3, 17 April 2009 (2009-04-17), pages 410-412, XP026080488, ISSN: 0167-5273, DOI: 10.1016/J.IJCARD.2007.11.096 [retrieved on 2008-01-29]
- None

## Description

### Field of the invention

The present invention relates to the fields of secretomes. More particularly, the invention relates to a method of deriving secretomes from mesenchymal stem cells.

### Background

Mesenchymal stem cells, or MSCs, are multipotent stromal cells which have the potential to differentiate into a variety of mesenchymal cell types of the adipocytic, chondrocytic and osteocytic lineages, including: osteoblasts, chondrocytes, neurons, muscle cells and adipocytes. This potential has been documented in specific cells and tissues in vivo and in vitro. MSCs are distributed in mammals all over the body and are responsible for regeneration. Commonly used tissues for the isolation of MSC are bone marrow, umbilical cord, cord lining and - increasingly - adipose tissue, which has a superior amount of MSCs.

Dendritic cells, or DCs, are antigen-presenting cells (APCs) which play a critical role in the regulation of the adaptive immune response. Dendritic cells are unique APCs and have been referred to as "professional" APCs, since the principal function of DCs is to present antigens, and because only DCs have the ability to induce a primary immune response in resting naive T-lymphocytes. To perform this function, DCs are capable of capturing antigens, processing them, and presenting them on the cell surface along with appropriate co-stimulation molecules. DCs also play a role in the maintenance of B-cell function and recall responses. Thus, DCs are critical in the establishment of immunological memory. The function of DCs falls broadly into three categories, each of which involve antigen presentation. The first category of DCs function is antigen presentation and activation of T-cells. The second category of DC function is not as well established, but it has been suggested that a different class of DCs exist with the function of inducing and maintaining immune tolerance. The third category of DCs, known as follicular DCs, appear to work to maintain immune memory in tandem with B-cells. DCs can be generated by transforming peripheral blood monocytes.

All cells communicate and exchange information by different ways, including the secretion of soluble factors, the cell-to-cell adhesion contact, and the intercellular exchange of organelles. The secretome of mesenchymal stem cells includes paracrine substances, exosomes and microvesicles. Over 150 paracrine substances, also called cytokines and chemokines, can be released by mesenchymal stem cells. Two distinct populations of vesicles with peculiar membrane structure, mechanism of production, pathophysiological relevance, and different size have been described: exosomes and microvesicles. Microvesicles and exosomes contain biomolecules, including messenger RNA and micro RNA. Exosomes and the whole secretome have been used for therapies in regenerative medicine to treat various illnesses such as osteoarthritis, cardiovascular diseases, neurological diseases, pulmonary diseases, diabetes and many more (see e.g. Bo Yu, et al., Exosomes Derived from Mesenchymal Stem Cells. Int. J. Mol. Sci. 15, 4142-4157, 2014; Eun Kyoung Jun et al., Hypoxic Conditioned Medium from Human Amniotic Fluid-Derived Mesenchymal Stem Cells Accelerates Skin Wound Healing through TGF-β/SMAD2 and Pl3K/Akt Pathways. Int. J. Mol. Sci. 15, 605-628, 2014; Yi Lee et al., Exosomes and microvesicles: extracellular vesicles for genetic information transfer and gene therapy. Human Molecular Genetics. Vol. 21, 2012, Review Issue; Imhof A. and Heinzer I., Continuous Monitoring of Oxygen Concentrations in Several Systems for Cultivation of Anaerobic Bacteria. J of Clin. Microbiol., 1646-1648, 1996). In the known therapies success rate and duration has been very variable, mainly due to the insufficient amount of proteins and RNA used in the studies.

The secretome of both MSCs and DCs consists of various proteins, RNAs and extracellular vesicles, such as, but not limited to, exosomes and microvesicles.

### 1. Introduction to exosomes and microvesicles

Cells communicate and exchange information by different ways, including the secretion of soluble factors, the cell-to-cell adhesion contact, and the intercellular exchange of organelles through nanotubular structures (Rustom A. et al; Nanotubular highways for intercellular organelle transport, Science 303:1007-1010, 2004). Recent studies have proposed that cell-derived small circular membrane vesicles, called exosomes and microvesicles, represent an additional mechanism of cell-to-cell communication by transfer of membrane components as well as the cytoplasmic content (Ratajczak J. et al., Membrane-derived microvesicles: important and underappreciated mediators of cell-to-cell communication, Leukemia 20:1487-1495, 2006; Camussi G. et al., Exosomes/microvesicles as a mechanism of cell-to-cell communication, Kidney Int. 78:838-848, 2010; Denzer K. et al., Exosome: from internal vesicle of the multivesicular body to intercellular signaling device, J. Cell Sci. 113, Pt 19:3365-3374, 2000). Two distinct populations of vesicles with peculiar membrane structure, mechanism of production, pathophysiological relevance, and different size have been described. Membrane fragments of 30-100nm diameter derived from the endosomal membrane compartment after fusion of secretory granules with the plasma membrane are defined as exosomes. Once released, exosomes bind the recipient cells through receptor-ligand interactions or fuse with the target cell membrane transferring membrane components, including cell receptors (Ratajczak J. et al, see supra), and discharging the portion of cytosol segregated within their lumen into the cytoplasm of recipient cells (Cocucci E et.al., Shedding microvesicles: artefacts no more, Trends Cell Biol. 19:43-51, 2009). The molecular cargo content of exosomes derives from active packaging of certain nucleic acid species leading to the presence of mRNAs in exosomes that are not found in donor cells (Valadi H. et al., Exosome-mediated transfer of mRNAs and microRNAs is a novel mechanism of genetic exchange between cells, Nat. Cell Biol. 9:654-659, 2009). Furthermore, relatively large microvesicles (100nm - 1 µm diameter) are formed from the surface membrane of activated cells in a calcium and calpain dependent manner following a disordered function of phospholipid transporters that results in the budding of altered membrane that exposes phosphatidylserine in the outer leaflet. Microvesicles and exosomes contain biomolecules, including mRNA and microRNA (miRNA or miR), packaged in a random process and their release is considered an expression of a pathological process in place. Molecular transfer from microvesicles and exosomes contributes to changes in the maturation and differentiation of target cells as for example microvesicles and exosomes released by endothelial progenitor cells trigger neo-angiogenesis in endothelial cells (Deregibus M.C. et al, Endothelial progenitor cell derived microvesicles activate an angiogenic program in endothelial cells by a horizontal transfer of mRNA, Blood 110:2440-2448, 2007).

### 2. Introduction to paracrine substances

MSCs secrete numerous growth factors and cytokines. A typical MSC secretion profile comprises growth factors, cytokines, ECM proteases, hormones, and lipid mediators. Paracrine signalling is a form of cell-cell communication in which a cell produces a signal to induce changes in nearby cells, altering the behavior or differentiation of those cells. Signalling molecules known as paracrine factors diffuse over a relatively short distance (local action), as opposed to endocrine factors (hormones which travel considerably longer distances via the circulatory system), juxtacrine interactions, and autocrine signalling. Cells that produce paracrine factors secrete them into the immediate extracellular environment. Factors then travel to nearby cells in which the gradient of factor received determines the outcome. However, the exact distance that paracrine factors can travel is not certain. Although paracrine signalling elicits a diverse array of responses in the induced cells, most paracrine factors utilize a relatively streamlined set of receptors and pathways. In fact, different organs in the body - even between different species - are known to utilize a similar sets of paracrine factors in differential development. The highly conserved receptors and pathways can be organized into four major families based on similar structures: Fibroblast growth factor (FGF) family, Hedgehog family, Wnt family, and TGF-β superfamily. Binding of a paracrine factor to its respective receptor initiates signal transduction cascades, eliciting different responses.

### 3. Introduction to manipulation and preconditioning of MSCs

Due to the potential of MSCs and secretomes obtained therefrom for therapeutic applications, various strategies have been proposed to obtain increased production or secretion of paracrine factors as described above.

Previous studies suggested that physiological preconditioning such as a hypoxic condition, wherein oxygen levels are reduced below physiological or normal conditions, promotes self-renewal of undifferentiated mesenchymal stem cells and enhances therapeutic potential. Hypoxic exposure activates several signal transduction pathways including hypoxia inducible factor (HIF), a master transcription factor that regulates the expression of hundreds of genes to promote cellular adaptation to the hypoxic condition. HIF-1 a is a pivotal signalling molecule for hypoxia-mediated upregulation of bFGF and hepatocyte growth factor (HGF) secretion, whereas HIF-2a is a key signalling molecule for hypoxia-mediated upregulation of VEGF secretion from MSCs. HIF-1a is widely expressed in almost all tissues, whereas the expression of HIF-2a is restricted to certain cell types such as vascular endothelial cells.

Other studies have focused on genetic manipulation of MSCs, whereby MSCs have been engineered with transgenes for conditional gene expression with the aim of improving cell survival and controlling the MSC secretome post-transplantation. Yet further studies have aimed at controlling MSC paracrine secretion through molecular preconditioning using compounds ranging from proteins such as cytokines, chemokines and growth factors to small molecule libraries. However, despite promising results, neither strategy has been able to address all requirements and there remains still a need for improved methods for the production of secretomes.

Past and potential future applications of proteomic techniques in mesenchymal stem cell secretomics, have been reviewed by e.g. Skalnikova et al (Biochimie 2013, 95, 2196). Other reviews report on the understanding of the mesenchymal stem cell secretome as a therapeutic for treatment of ischemic heart disease (Ranganath et al; Cell Stem Cell 2012, 10, 244). It was further suggested (Chang et al; Clin Sci 2013, 124, 165) that mesenchymal stem cells secrete bioactive factors, including HGF and VEGF, and thus may show therapeutic potential in experimental TBI by stimulating neurogenesis. Other prior art in the area includes reports on the impact of normoxic and hypoxic cell-culture conditions on the expression and secretion of bone marrow-derived mesenchymal stem cell-derived paracrine molecules, and the use of mesenchymal stem cell-derived secretome and micovesicles as cell-free therapeutics for neurodegenerative diseases (Chen et al, PLOS ONE 2014, 9, e96161 ); on the use of mesenchymal stem cells cultured in the presence of phenanthroline for treating diseases related to angiogenesis (EP 2 607 478); on culturing neural stem cells under hypoxic conditions to obtain neural stem cell exosomes or microvesicles, which may be used in therapy (WO 2013/150303); on anoxic preconditioning to enhance the capacity of mesenchymal stem cells to repair infarcted myocardium (Wang et al, Science 2009, 133, 410); and on the influence of oxygen levels on CD34+ cells present in apheresis components (Ivanovi et al, Transfusion 2000, 40, 1482).

### SUMMARY OF THE INVENTION

According to a first aspect of the present invention, a method of producing a secretome secreted by a mesenchymal stem cell comprising at least one biological property of a mesenchymal stem cell is provided, wherein the cells are kept during at least one period of at least 5 minutes under anoxic conditions of 0%.

Preferably the at least one period of anoxic conditions is followed by a subsequent period of keeping the cells under oxic conditions and/or hypoxic conditions (hereinafter referred to as a cycle). In preferred embodiments the cells are subjected to at least one cycle, preferably more than one, such as two to four cycles.

Surprisingly, it was found that at least one, i.e. one or repeated exposure to such stress conditions had a significant positive influence on the amount and quality of exosomes, microvesicles and/or paracrine substances secreted by mesenchymal stem cells or secreted by the dendritic cells.

The secretome or secretome vesicles or paracrine secretome substances secreted by a mesenchymal stem cell comprising at least one biological property of a mesenchymal stem cell, which are produced according to the present invention, and more specifically a pharmaceutical composition thereof together with a pharmaceutically acceptable carrier, excipient or diluent, may be suitable for use in a method of treating a disease. Such use may be in a method of autogenous medical treatment of a disease or autogenous cosmetic treatment in an individual (which acted as donor for the mesenchymal stem cell or the dendritic cell used for producing the secretome or secretome vesicle or paracrine substance). The disease may be selected from the group consisting of: osteoarthritis, cardiovascular diseases, lung diseases, liver diseases, neurodegenerative diseases including multiple sclerosis, Parkinson, Alzheimer and Graft-versus-host-disease (GVHD) or Crohn's disease. The secretome or secretome vesicle or paracrine secretome substance may be used to aid wound healing, scar reduction or cartilage- or bone-formation.

The practice of the present invention will employ, unless otherwise indicated, conventional techniques of chemistry, molecular biology, microbiology, recombinant DNA and immunology, which are within the capabilities and the general knowledge of a person of ordinary skill in the art. Such techniques are explained in the literature (see, for example, Gstraunthaler G. and Lindl T., Zell- und Gewebe-kultur: Allgemeine Grundlagen und spezielle Anwendungen. 7. Ed. 2013, XIII, Heidelberg: Spektrum Akademischer Verlag; Øystein B, Cell culture technology: Recent advances and future prospects, 2012, Euroscicon Meeting Reports Book 1, Astrid E. (Ed)). Honnao Publishing; Lanza R. P, Klimanskaya I. (Eds)., 2009, Essential Stem Cell Methods (Reliable Lab Solutions). Academic Press)

### BRIEF DESCRIPTION OF THE FIGURES

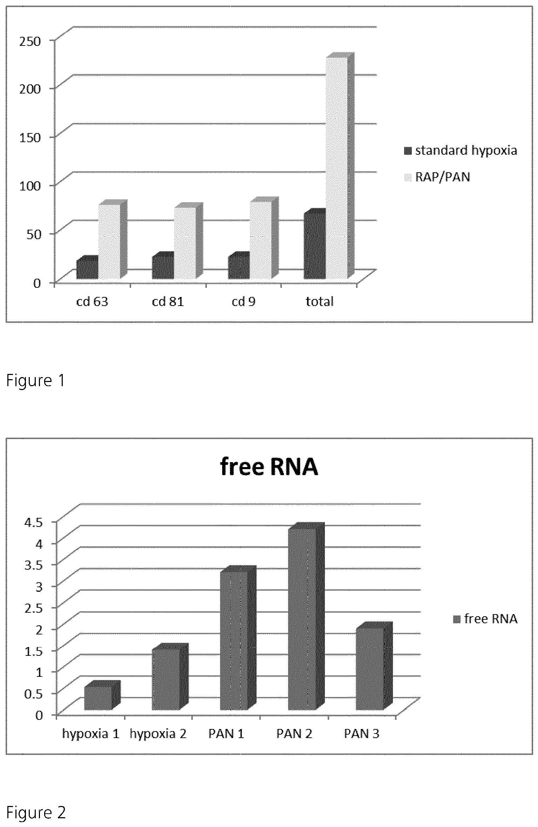

- Figure 1: shows in a bar graph the semiquantitatively measured (ocular density (OD) measurement in photospectrometer) amounts of surface markers CD 63, 81 and 9 via RAP/PAN technology compared with standard hypoxic conditions and the total indicating a 30fold increase;
- Figure 2: shows a comparison of free RNA content of secretome produced under standard hypoxia and the PAN system according to Example 1 (Values in ng/ul measured by Maestro Nano photometer (y-axis));

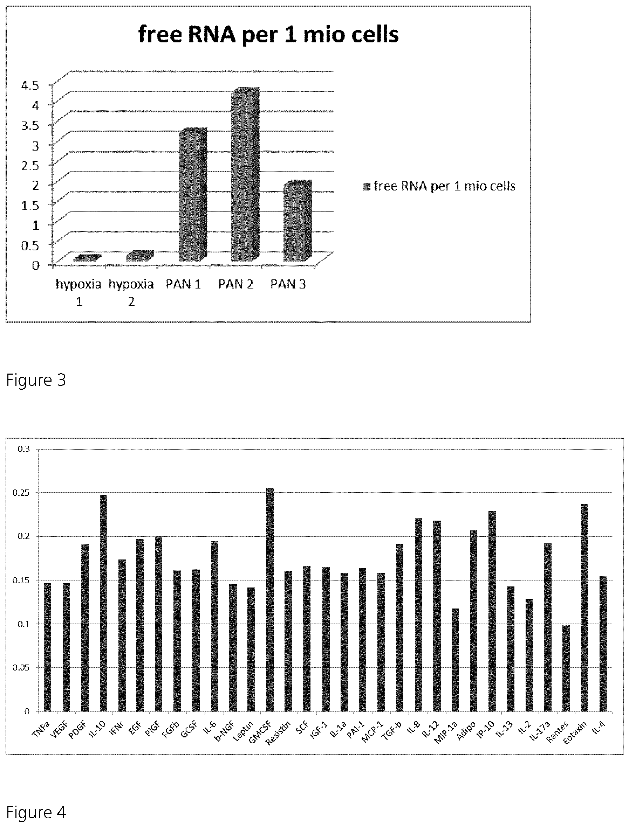

- Figure 3: shows a comparison between free RNA in secretome produced by 1 x 10⁶ MSCs under standard hypoxia and 1 x 10⁶ MSCs with PAN technology according to Example 1 (Values in ng per million cells measured by Maestro Nano photometer (y-axis));
- Figure 4: shows the amount of cytokines measured (ocular density (OD) measurement in photospectrometer) after secretome production; y-axis: OD at 450 nm Cytokine Elisa Assay
- Figure 5: shows the carbondioxide/temperature/oxygen curves during a 120minute anoxic period (x-axis: time in minutes; y-axis: temperature in degrees celcius, CO₂ in %, 02 in %);
- Figure 5a: shows the single carbondioxide curve according to Figure 5 (Values in % gas on y-axis and time in minutes on x-axis);
- Figure 5b: shows the single temperature curve according to Figure 5 (Values in degrees celsius on y-axis and time in minutes on x-axis); and
- Figure 5c: shows the oxygen curve according to Figure 5. (Values in % gas on y-axis and time in minutes on x-axis).

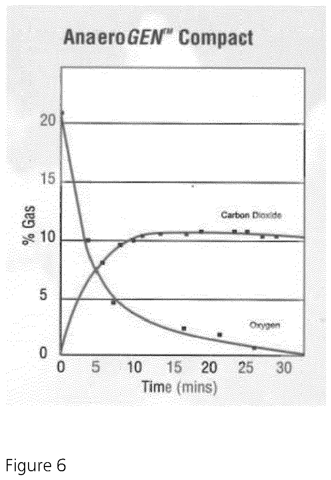

- Figure 6: shows an oxygen/carbondioxide curve according to manufacturer of AnaeroGEN compact AnaerobJar.
- Figure 7: is a Volcano plot showing the miRNA expression differences between RAP/Anoxia secretome (top left) and PAN/Hypoxia secretome (bottom right) according to Example 3. The lighter (or grey) dots represent miRNA with little differences regarding their signal strength in the Affymetrix expression profile and the darker (or blue) dots represent miRNA with an at least twofold difference in signal strength.
- Figure 8: represents a further volcano diagram of the results obtained according to Example 3, showing miRNA differing by up to a 16 fold signal strength.

### Detailed description of the invention

The following definitions apply unless stated otherwise:
"Oxic conditions" refers in the present description to ambient atmospheric oxygen tension of approximately 5-21 % O₂ (volume:volume; 1'013 hPa; 50'000 - 210'000 parts per million oxygen).

"Hypoxic conditions" (or "standard hypoxia" conditions) refers to oxygen tensions which are lower than oxic conditions, i.e. below physiologic levels, preferably between approximately 1-5 % 02 (10'000 - 50'000 ppm oxygen).

"Anoxic conditions" refers to oxygen tensions of about 0%, preferably 0%. Anoxic conditions may be created in several approaches. Each approach may be used independently or in combination with one or more other approaches. In one embodiment, anoxic conditions can be achieved by reducing the oxygen level below the normal level to about 0%.

The term "lactate" or "isotonic lactate solution" refers to a concentration of lactic acid or the lactate anion in the range of about 10 to about 500 mmol. A preferred isotonic lactate solution is "Ringer's lactate solution" which refers to a solution of about 130 mmol/I sodium ion, 109 mmol/I chloride ion, 28 mmol/I lactate, 4 mmol/I potassium ion, and 1.5 mmol/I calcium ion in purified water.

The term "cycle" refers to a period under anoxic conditions followed by a period under oxic and/or hypoxic conditions. In one embodiment, a cycle comprises a period under anoxic conditions followed by a period under oxic conditions. In another embodiment a cycle comprises a period under anoxic conditions followed by a period under hypoxic conditions followed by a period of oxic conditions.

Preferably MSCs are thawed and cultivated and DCSs are thawed in cell culture flasks with a special cell medium (nutrient). According to a first preferred method (hereinafter called RAP system) the cells are then detached and washed three times in warm phosphate buffered saline (PBS) or other saline solutions. According to a second preferred method the cells are washed directly in the cell culture flasks without detachment (hereinafter called PAN system).

After washing according to either one of the above mentioned systems (RAP or PAN) 2 to 15 ml of warm PBS are added and the cells are kept for at least one period of at least 5 minutes under anoxic conditions created by oxygen depletion (i.e. in an anaerobe jar) before leaving the cells at oxic conditions at room temperature or at 37°C for a total of between 5 and 24 hours.

The duration of the period under anoxic conditions is at least 5 minutes such as between 5 minutes and 24 hours. The duration of the period under anoxic conditions created by oxygen depletion lies preferably in a range of between 5-120 minutes, such as 5 min, 20 min, 40 min, 60 min, 90 min or 120 min. According to further preferred embodiments of the present invention the cells are exposed to more than one period under anoxic conditions, i.e. the above cycle of anoxic conditions (i) or (ii) followed by oxic conditions is repeated. Preferably the cells are exposed to 2 to 4 periods of anoxic conditions (i) or (ii), each followed by a subsequent period of keeping the cells under oxic conditions. During the anoxic conditions obtained by oxygen depletion (according to (i)), the oxygen concentration is about 0%, preferably 0%.

In another preferred embodiment, the cells are exposed to both anoxic conditions (i) and (ii), e.g. first to a period of anoxic conditions according to (ii) followed by a period of anoxic conditions according to (i), preferably followed by a subsequent period of keeping the cells under oxic conditions.

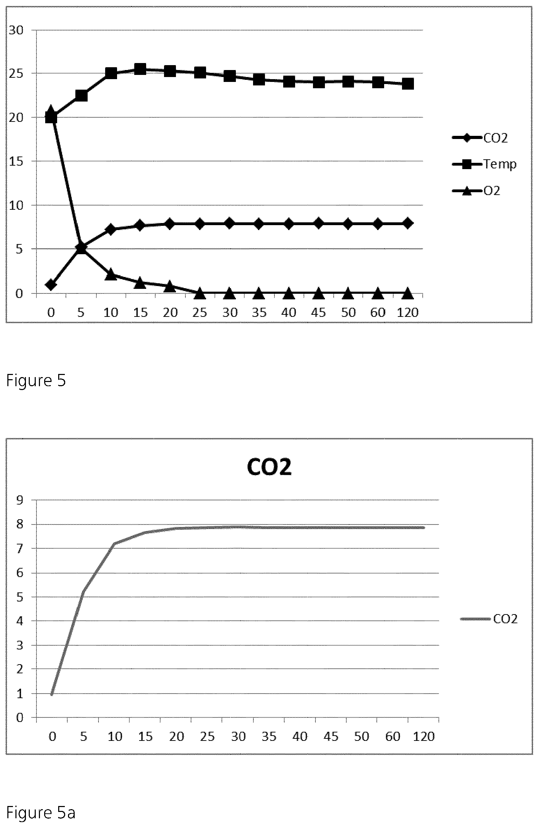

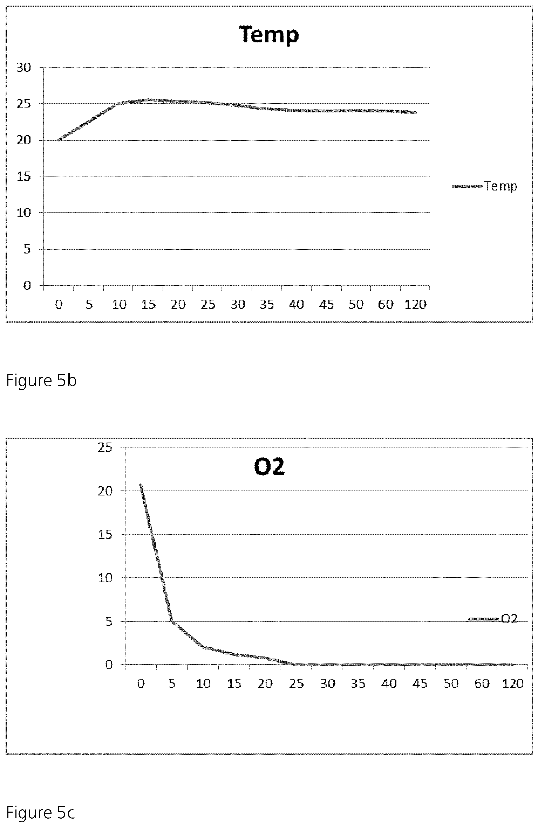

Reduction of oxygen content according to anoxic conditions (i) may be achieved using any Atmosphere Generation System for culturing the cells under anoxic conditions in an enclosed volume, such as an Oxoid Anaerogen system, a Hypoxia Incubator Chamber produced by STEMCELL Technologies SARL (40 Rue des Berges, Miniparc Polytec, Bâtiment Sirocco, 38000 Grenoble, France), or any other Anaerobic Chamber. It is clearly visible from Figure 5, that it takes several minutes in the experimental setup as described herein to reach the anoxic conditions by oxygen depletion, i.e. an oxygen tension of about 0%, such as 0%. Accordingly, it is understood that the cells are exposed to normoxic conditions and then hypoxic conditions before the system reaches anoxic conditions of about 0%, preferably 0% oxygen. In the experimental setup used by the inventor according to the present invention, it takes about 25 minutes to reach anoxic conditions.

The secretome is then harvested for example by simple aspiration of the buffer solution, such as PBS or Ringer's lactate. The secretome is then preferably centrifuged to discard cells and afterwards microfiltrated to include or exclude various components of the secretome.

The secretomes may be analyzed by known methods, such as Western blotting, enzyme-linked immunosorbent assay, as well as (mass spectrometry-based) proteomics approaches as described in the literature, and includes determining the expression profile of miRNA regulating gene expression at the posttranscriptional level and mRNA as regenerative messengers at cell signalling level. Further analysis including isolation of single components and characterization thereof may be performed before or after testing for their in vitro immunomodulatory effect.

Compared to previously known systems exposing MSC cells to stress conditions according to the invention the novel production method results in a high (e.g. 30 fold) increase in the amount of RNA in the secretome.

A further advantage is the high amount of miR and HIF1 a (hypoxia inducible factor 1 alpha) in the secretome after the anoxic treatment. For example, miR-22 which can be used in a method of treating cardiovascular diseases, miR-133b for neurological diseases, miR-146 for wound healing, miR-204 for pulmonary diseases. Thereby the present invention allows the person skilled in the art to produce secretome specifically for certain diseases. In particular, clinical grade secretome for therapies in regenerative medicine for a host of diseases such as osteoarthritis, cardiovascular diseases, lung diseases, liver diseases, neurodegenerative diseases including multiple sclerosis, Parkinson, Alzheimer, GVHD, Crohn's disease and many more.

All of the above said is not only true for secretome, but also for secretome vesicles, in particular for exosomes and/or microvesicles, and for paracrine secretome substances. The person skilled in the art knows how to purify, extract etc. said vesicles and substances from the whole secretome for example by utilizing the teachings from the literature, see Graça R. and Stoorvogel, W., Extracellular vesicles: Exosomes, microvesicles, and friends, J. Cell Biol., Vol. 200 No. 4 373-383, 2013; Tauro BJ et al, Comparison of ultracentrifugation, density gradient separation, and immunoaffinity capture methods for isolating human colon cancer cell line LIM1863-derived exosomes, Methods, 56(2):293-304, 2012.

### Example 1 (PAN System/Anoxic conditions through O₂ depletion)

According to a first example secretome is produced by the following steps:
1.1. Fill a precoated T75 culture flask with 15 ml of SMP alpha minus stem cell medium and transfer into incubator for 30-120 minutes. Then spray culture flask with 70% IPA and transfer to laminar air flow.
1.2. Thaw a cryopreserved vial with approximately 1 mio MSC by removing it from the liquid nitrogen storage tank and transfer it immediately into the warm water bath preheated to 37-40 °C for 1-4 minutes. Remove vial from bath immediately after last bit of solid ice has been thawed
1.3. Spray vial with 70% alcohol and transfer to laminar air flow. Pipette MSCs with 2ml serological pipette into the ready culture flask and swirl gently and transfer to incubator (5% CO₂ and 37°C)
1.4. Pipette 15 ml of SMP alpha minus stem cell medium into a 50 ml centrifuge tube and transfer to water bath for at least 30 minutes.
1.5. Change stem cell media 2-8 hours after transfer of vial into cell culture flask. Verify that at least 90% of the cells have settled to the plastic floor.
1.6. Leave culture flask in the incubator for 1 to 3 days until a cell confluence of 75%-90% has been achieved.
1.7.a. Transfer flask to laminar air flow bench after spraying with 70% IPA and remove supernatant. Wash Flask with warm Dulbecco's PBS or similar three times with 10 ml each carefully. Cover the cells with 10 ml of a saline solution such as PBS.
1.8.a. Prepare the AnaerobJar Oxoid system by spraying with 70% IPA and leaving to dry in the laminar air flow.
1.9.a. Place the culture flask into the lying Anaerobjar, insert the oxygen capture sachet and close the lid of the jar. Leave for 1 hour in the laminar air flow bench at room temperature, then open the jar carefully and place flask in the CO₂ incubator or at room temperature in the bench for 4 hours.

The process under 1.9. can be repeated immediately for additional cycles of anoxia. Or the secretome containing saline can be removed and replaced by culture medium such as SMP alpha minus for 1 to 48 hours before recommencing the cycle described under 1.9.

The whole cycle under 1.9.can also be performed in the incubator at 37 °C instead of room temperature.

Also, after the initial decrease of oxygen over approximately 30 minutes to anoxia at 0% 02 this anoxia phase can be shortened to 5 minutes or prolonged to 12 hours. After prolonged anoxia over 2 hours the cells will not be viable anymore for subsequent cycles.

Also, after the initial anoxia phase, cells can be kept in saline for more than 4 hours, i.e. up to 48 hours.

1.7.b.-1.9.b. Instead of the anaerobe jars a CO₂/O₂ incubator or an alternative device for culturing the cells under anoxic conditions in an enclosed volume can be used (e.g. a Hypoxia Incubator Chamber by STEMCELL Technologies SARL) or other newly designed anoxia systems.

1.10. Remove the saline under sterile conditions under the laminar air flow and centrifuge at 300 G for 3 minutes to remove detached stem cells. If only exosomes are needed for the therapy, filtrate the saline afterwards through a 0.45 µm syringe filter to remove microvesicles. Microvesicles can be retrieved per reverse washing of the filter.

1.11. Transfer saline into a glass vial for immediate application or store at - 80 °C in a RNase free plastic centrifuge tube.

1.12. Add 15 ml of SMP alpha minus stem cell medium to the culture flask and return it to the incubator.

1.13. Leave in incubator, with media changes every 2-3 days if necessary until cells have regrown to 80% confluence before repeating the harvesting procedure or freezing them according the standard procedures in liquid nitrogen.

### Material und Devices

### 1 Reagents and Solvents

(1) SMP alpha minus
(2) PBS Dulbecco or similar w/o Ca/Mg
(3) Optionally Ringer's lactate or
(4) Optionally other saline approved for use in humans

### 2 Devices

(1) CO2 Incubator / CO₂/O₂ incubator
(2) Laminar flow bench
(3) Centrifuge
(4) Water bath 35 ± 4°C
(5) Pipetting aid and serological pipettes
(6) Inverted Microscope Bresser
(7) Anaerob Jar or similar system
(8) Precoated culture flasks or self coated flasks
(9) Centrifuge tubes, RNase free

### Example 2 (RAP System/Anoxic conditions through O₂ depletion)

According to a second example secretome is produced by the following steps:
2.1. Fill a precoated T75 culture flask with 15 ml of SMP alpha minus stem cell medium and transfer into incubator for 30-120 minutes. Then spray culture flask with 70% IPA and transfer to laminar air flow.
2.2. Thaw a cryopreserved vial with approximately 1 mio MSC by removing it from the liquid nitrogen storage tank and transfer it immediately into the warm water bath preheated to 37-40 °C for 1-4 minutes. Remove vial from bath immediately after last bit of solid ice has been thawed
2.3. Spray vial with 70% alcohol and transfer to laminar air flow. Pipette MSCs with 2ml serological pipette into the ready culture flask and swirl gently and transfer to incubator (5% CO₂ and 37°C)
2.4. Pipette 15 ml of SMP alpha minus stem cell medium into a 50 ml centrifuge tube and transfer to water bath for at least 30 minutes.
2.5. Change stem cell media 2-8 hours after transfer of vial into cell culture flask. Verify that at least 90% of the cells have settled to the plastic floor.
2.6. Leave culture flask in the incubator for 1 to 3 days until a cell confluency of 75%-90% has been achieved.
2.7. Subcultivate the MSCs to 3 T75 and then again to 9 T75 flasks to achieve around 8 mio cells. Repeat expansion process accordingly to achieve higher cell amounts.
2.8. Detach stem cells when the required amount of cells and confluence has been achieved. This can be done by scraping or enzymatic detachment.
2.9. Centrifuge the detached cells and wash thrice with 10ml warm Dulbecco's PBS or similar saline solution by resuspending the cells in new PBS and discarding the supernatant after centrifugation.
2.10. Resuspend the cells in 2-20 ml Ringer's lactate, Dulbecco's PBS or similar saline solution.
2.11.a. Prepare the Anaerobe Jar Oxoid system by spraying with 70% isopropanol (IPA) and leaving to dry in the laminar air flow.
2.12.a. Place the Centrifuge tube with the MSC suspension into the standing Anaerobe jar, insert the oxygen capture sachet and close the lid of the jar. Leave for 1 hour in the laminar air flow bench at room temperature, then open the jar carefully and place tube in the CO₂ incubator or at room temperature in the bench for 4 hours.
2.11 .b.-2.1 2.b. As an alternative to the anaerobe jars a CO₂/O₂ incubator or an alternative device for culturing the cells under anoxic conditions in an enclosed volume can be used (e.g. a Hypoxia Incubator Chamber by STEMCELL Technologies SARL) or other newly designed anoxia systems.
2.12.c. The process under 2.12.a can be repeated immediately for additional cycles of anoxia as described under 2.12.a. Or the secretome containing saline can be harvested and replaced by culture medium such as SMP alpha minus for 1 to 48 hours before recommencing the cycle.

The whole cycle under 2.12.a can also be performed in the incubator at 37 °C instead of room temperature.

Also, after the initial decrease of oxygen over approximately 30 minutes to anoxia at 0% 02 this anoxia phase can be shortened to 5 minutes or prolonged to 12 hours. After prolonged anoxia over 2 hours the cells will not be viable anymore for subsequent cycles.

Also, after the initial anoxia phase, cells can be kept in saline for more than 4 hours, i.e. up to 48 hours.

2.13. Remove the saline under sterile conditions under the laminar air flow and centrifuge at 300 G for 3 minutes to remove detached stem cells. If only exosomes are needed for the therapy filtrate the saline afterwards through a 0.45 µm syringe filter to remove microvesicles. Microvesicles can be retrieved per reverse washing of the filter.

2.14. Transfer saline into a glass vial for immediate application or store at - 80 °C in a RNase free plastic centrifuge tube.

2.15. Add 15 ml of SMP alpha minus stem cell medium to resuspend the cells in the centrifuge flask and then place cells into the culture flask and return it to the incubator.

2.16. Leave in incubator, with media changes every 2-3 days if necessary until cells have regrown to 80% confluence before repeating the harvesting procedure or freezing them according the standard procedures in liquid nitrogen.

### Material und Devices

### Reagents and Solvents

(1) SMP alpha minus
(2) PBS Dulbecco or similar without Ca/Mg
(3) Optionally Ringer's lactate
(4) Optionally other saline approved for use in humans
(5) Cell Dissociation Reagent such as Accutase®

Devices used in Example 2 are identical to devices according to example 1.

According to a further embodiment, step 2.12a is performed with a 2 hour exposure in the Anaerobe Jar under anoxic conditions followed by a 1 hour break at normoxic conditions. This cycle is repeated 2-4 times.

Duration of the production is for example 5 hours, 12 and 24 hours.

### Example 3: Comparative analysis of miRNA production in secretomes produced under anoxic vs. hypoxic conditions

A first set of secretomes was produced using the PAN-procedure in combination with hypoxic conditions (PAN/Hypoxia; 1 % oxygen, 4h) by the following steps

Steps 1.1 - 1.6 were performed according to Example 1.

1.7. Transfer flask to laminar air flow bench after spraying with 70% IPA and remove supernatant. Wash Flask with warm Dulbecco's PBS or similar three times with 10 ml each carefully. Cover the cells with 10 ml of a saline solution such as PBS.

1.8. Prepare the anaerobe jar by spraying with 70% IPA and leaving to dry in the laminar air flow.

1.9. Place the culture flask into the anaerobe jar and close the lid of the jar. Leave for 30 minutes, then carefully open the anaerobe jar and close the screw cap of the culture flask and leave in the laminar air flow bench at room temperature and 1% oxygen for 4 hours

1.10. Remove the saline under sterile conditions under the laminar air flow and centrifuge at 300 G for 3 minutes to remove detached stem cells. If only exosomes are needed for the therapy, filtrate the saline afterwards through a 0.45 µm syringe filter to remove microvesicles. Microvesicles can be retrieved per reverse washing of the filter.

1.11. Transfer saline into a glass vial for immediate application or store at - 80 °C in a RNase free plastic centrifuge tube.

1.12. Add 15 ml of SMP alpha minus stem cell medium to the culture flask and return it to the incubator.

1.13. Leave in incubator, with media changes every 2-3 days if necessary until cells have regrown to 80% confluence before repeating the harvesting procedure or freezing them according the standard procedures in liquid nitrogen.

A second set of secretomes was produced using the RAP-procedure in combination with anoxic conditions (RAP/Anoxia; 0% oxygen for 30 min) followed by a period of hypoxic conditions (1 % oxygen for 4 hours) by the following steps:

Steps 2.1 - 2.10 were performed according to example 2.

2.11. Prepare the AnaerobJar Oxoid system by spraying with 70% isopropanol (IPA) and leaving to dry in the laminar air flow.

2.12a. For the anoxic period of 30 min at 0% 02, place the centrifuge tube with the MSC suspension into the standing Anaerobjar, insert the oxygen capture sachet and close the lid of the jar. Leave for a total of 1 hour in the laminar air flow bench at room temperature (placement of the centrifuge tube with loose caps achieved reduction of ambient 02 concentration to 0% O₂ within 30 minutes. Cells where then kept at anoxia for further 30 minutes before carefully opening the Anaerobe Jar). Then open the jar carefully and place tube in the CO₂ incubator or at room temperature in the bench under the laminar air flow with loose cap for 4 hours (at 1% oxygen) before harvesting the secretome.

2.13. Remove the saline under sterile conditions under the laminar air flow and centrifuge at 300 G for 3 minutes to remove detached stem cells. If only exosomes are needed for the therapy filtrate the saline afterwards through a 0.45 µm syringe filter to remove microvesicles. Microvesicles can be retrieved per reverse washing of the filter.

2.14. Transfer saline into a glass vial for immediate application or store at - 80 °C in a RNase free plastic centrifuge tube.

2.15. Add 15 ml of SMP alpha minus stem cell medium to the culture flask and return it to the incubator.

2.16. Leave in incubator, with media changes every 2-3 days if necessary until cells have regrown to 80% confluence before repeating the harvesting procedure or freezing them according the standard procedures in liquid nitrogen.

The secretome was analysed for miRNA by using the Qiagen EN-RNeasy Kit and spin columns for isolation and extraction of miRNA. Briefly, tissue samples are homogenized in QIAzol Lysis Reagent. After addition of gDNA Eliminator Solution and chloroform, the homogenate is separated into aqueous and organic phases by centrifugation. RNA partitions to the upper, aqueous phase, while DNA partitions to the interphase and proteins to the lower, organic phase or the interphase. The upper, aqueous phase is collected, and RNA is purified using RNeasy spin columns. The upper, aqueous phase is mixed with ethanol to provide appropriate binding conditions and applied to an RNeasy Mini spin column. Total RNA binds to the spin column membrane, and phenol and other contaminants are efficiently washed away. High-quality RNA is then eluted in RNase-free water.

After isolation, miRNA was analysed for quality control reasons using the Agilent Bioanalyzer 2100 RNA 6000 Nano Assay regarding validation of total RNA. This analysis produces both a gel-like image as well as electrophoretic data and monitors indications of RNA degradation such as decreasing ratio of ribosomal bands, additional peaks below the ribosomal bands, decrease in overall RNA signal, shift towards shorter fragments and genomic contamination.

Concentration and sample quality of miRNA was then measured with the Nanodrop ND-1000 Spectrometer, analysing the 260/280 ratio indicating possible protein contamination (when below 1.8) and the 260/230 ratio indicating possible contamination with organic solvents (when below 1.6).

Finally, an electropherogram using the Agilent RNA pico labchip was performed. All three quality tests were considered valid and suitable for further analyses.

MiRNA expression was analysed with the Affymetrix GeneChip® miRNA 4.0 Array. Affymetrix' high-density array provides the most sensitive, accurate, and complete measurement of small non-coding RNA transcripts involved in gene regulation. These Affymetrix miRNA Arrays provide 100% miR Base v20 coverage, 30'424 mature miRNA (all organisms) and 5'214 human miRNA.

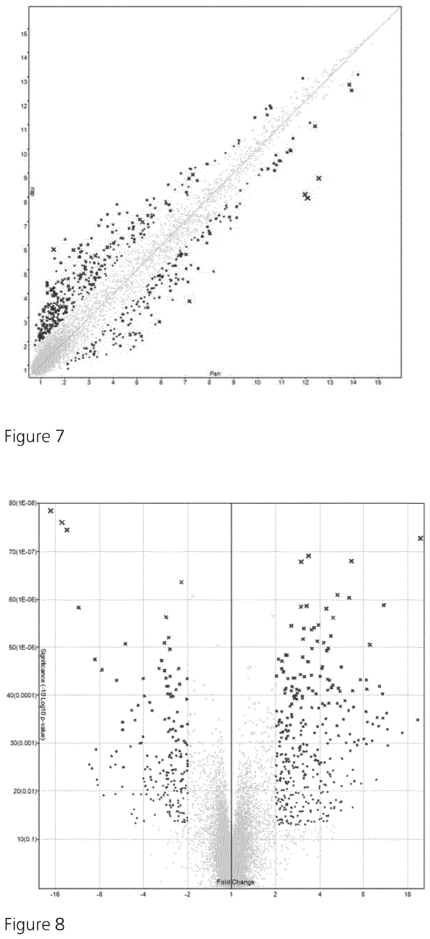

The miRNA expression analysis revealed significant differences in miRNA content when analysing the PAN/Hypoxia secretome with the RAP/Anoxia secretome. Due to the enormous amount of miRNA data generated with the Affymetrix expression profile, the changes are best viewed with the volcano setting (figure 7). This graphic was constructed by plotting the log 10 of the expression level of the RPA/anoxia secretome (i.e. signal strengths of measured RPA/anoxia miRNA production) on the y-axis against the log10 of the expression level of the PAN/hypoxia secretome on the x-axis (i.e. signal strengths of measured PAN/hypoxia miRNA production). Light (or grey) dots represent miRNA with little differences regarding their signal strength in the Affymetrix expression profile and dark (or blue) dots represent an at least twofold difference in signal strength, thus showing a clear and distinctive difference in miRNA expression profile of the PAN/Hypoxia versus the RAP/Anoxia secretome.

The extent of differences in signal strength is visualized in a second volcano diagram (figure 8). This graphic was constructed by plotting the negative log 10 of the p-value (significance) on the y-axis (with low p-values (highly significant) toward the top of the plot), against the log 10 of the fold change between the two conditions on the x-axis (with large magnitude fold changes being left- or right-of center). In Figure 8 the extent of differences in signal strength is clearly illustrated by some miRNA differing by a 16 fold signal strength.

The person skilled in the art can, based on the technical information provided above and utilizing his technical knowledge, upscale the production methods according to the examples provided above without inventive activity.

## Claims

1. A method of producing a secretome secreted by a mesenchymal stem cell comprising at least one biological property of a mesenchymal stem cell wherein the method comprises at least one period of keeping the cells at least 5 minutes under anoxic conditions, and wherein the anoxic conditions are achieved by keeping oxygen tensions of 0% 02.

2. The method according to claim 1, **characterized in that** the period of keeping the cells under anoxic conditions is followed by a period of keeping the cells under oxic and/or hypoxic conditions.

3. The method according to claim 2, **characterized in that** a cycle comprising a period of keeping the cells under anoxic conditions and a subsequent period of keeping the cells under oxic conditions is performed more than one time, preferably 2 to 4 times.

4. The method according to any of the preceding claims, **characterized in that** the period of keeping the cells under anoxic conditions lasts between 5 minutes to 24 hours.

5. The method according to any of the preceding claims, **characterized in that** exosomes, microvesicles and/or paracrine secretome substances are isolated separately or in groups from the secretome.

6. The method according to any of the preceding claims, wherein the cells are kept under anoxic conditions in an AnaerobJar, a CO₂/O₂ incubator or another device for culturing the cells under anoxic conditions.

## Patentansprüche

1. Verfahren zur Herstellung eines von einer mesenchymalen Stammzelle sezernierten Sekretoms, umfassend wenigstens eine biologische Eigenschaft einer mesenchymalen Stammzelle, wobei das Verfahren wenigstens einen Zeitraum umfasst, in dem die Zellen wenigstens 5 Minuten unter anoxischen Bedingungen gehalten werden, und wobei die anoxischen Bedingungen unter Beibehalten von Sauerstoffspannungen von 0% O₂ erreicht werden.

2. Verfahren nach Anspruch 1, **dadurch gekennzeichnet, dass** sich an den Zeitraum, in dem die Zellen unter anoxischen Bedingungen gehalten werden, ein Zeitraum anschließt, in dem die Zellen unter oxischen und/oder hypoxischen Bedingungen gehalten werden.

3. Verfahren nach Anspruch 2, **dadurch gekennzeichnet, dass** ein Zyklus, der einen Zeitraum, in dem die Zellen unter anoxischen Bedingungen gehalten werden, und einen anschließenden Zeitraum, in dem die Zellen unter oxischen Bedingungen gehalten werden, umfasst, mehr als einmal, bevorzugt 2- bis 4-mal, durchlaufen wird.

4. Verfahren nach einem der vorhergehenden Ansprüche, **dadurch gekennzeichnet, dass** der Zeitraum, in dem die Zellen unter anoxischen Bedingungen gehalten werden, zwischen 5 Minuten und 24 Stunden währt.

5. Verfahren nach einem der vorhergehenden Ansprüche, **dadurch gekennzeichnet, dass** Exosomen, Mikrovesikel und/oder parakrine Sekretomsubstanzen getrennt oder in Gruppen aus dem Sekretom isoliert werden.

6. Verfahren nach einem der vorhergehenden Ansprüche, wobei die Zellen unter anoxischen Bedingungen in einem AnaerobJar, einem CO₂/O₂-Inkubator oder einer anderen Vorrichtung zur Kultivierung der Zellen unter anoxischen Bedingungen gehalten werden.

## Revendications

1. Procédé de production d'un sécrétome sécrété par une cellule souche mésenchymateuse comprenant au moins une propriété biologique d'une cellule souche mésenchymateuse, le procédé comprenant au moins une période de maintien les cellules, d'au moins 5 minutes, dans des conditions anoxiques, et dans lequel les conditions anoxiques sont atteintes en maintenant les tensions d'oxygène à 0% de O₂.

2. Procédé selon la revendication 1, **caractérisé en ce que** la période de maintien les cellules dans des conditions anoxiques est suivie d'une période de maintien les cellules dans des conditions oxiques et/ou des conditions hypoxiques.

3. Procédé selon la revendication 2, **caractérisé en ce qu'**un cycle, comprenant une période de maintien les cellules dans des conditions anoxiques et une période consécutive de maintien des cellules dans des conditions oxiques, est exécuté plus d'une fois, de préférence de 2 à 4 fois.

4. Procédé selon l'une quelconque des revendications précédentes, **caractérisé en ce que** la période de maintien les cellules dans des conditions anoxiques dure de 5 minutes jusqu'à 24 heures.

5. Procédé selon l'une quelconque des revendications précédentes, **caractérisé en ce que** des exosomes, des microvésicules et/ou des substances paracrines du sécrétome sont isolés séparément ou en groupes du sécrétome.

6. Procédé selon l'une quelconque des revendications précédentes, dans lequel les cellules sont maintenues dans des conditions anoxiques dans un AnaerobJar, un incubateur à CO₂/O₂, ou un autre appareil destiné à cultiver les cellules dans des conditions anoxiques.
